(19) Europäisches Patentamt
European Patent Office
Office européen des brevets

(11) Publication number: 0 089 230 A1

# (12) EUROPEAN PATENT APPLICATION

(21) Application number: **83301426.9**

(22) Date of filing: **15.03.83**

(51) Int. Cl.$^3$: **G 03 C 1/06**
G 03 C 1/30, G 03 C 1/34
G 03 C 5/16, C 07 D 513/02

(30) Priority: **15.03.82 JP 40383/82**

(43) Date of publication of application:
**21.09.83 Bulletin 83/38**

(84) Designated Contracting States:
**DE FR GB**

(71) Applicant: **KONISHIROKU PHOTO INDUSTRY CO. LTD.**
**No. 26-2, Nishishinjuku 1-chome Shinjuku-ku**
**Tokyo 160(JP)**

(72) Inventor: **Machida, Katsutoshi**
**972-4 Yokokawa-cho**
**Hachioji-shi Tokyo 193(JP)**

(72) Inventor: **Sakuma, Haruhiko**
**65-4 Takakura-cho**
**Hachioji-shi Toyko 192(JP)**

(72) Inventor: **Ishikawa, Naooki**
**7-17 Takagi-cho**
**Kokubunji-shi Tokyo 185(JP)**

(72) Inventor: **Kurihara, Seiji**
**2-5 Ohwada-cho**
**Hachioji-shi Tokyo 192(JP)**

(74) Representative: **Ellis-Jones, Patrick George Armine et al,**
**J.A. KEMP & CO. 14 South Square Gray's Inn**
**London WC1R 5EU(GB)**

## (54) Silver halide photographic material.

(57) A silver halide photographic material comprising a support and one or more layers, at least one layer containing a compound of the following formula (I) and at least one compound selected from among the compounds of the following formulae (II), (III) and (IV) is disclosed:

Formula (I)

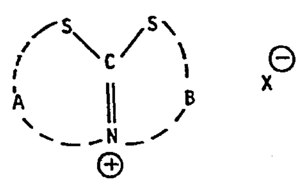

(wherein A and B each represent non-metallic atoms necessary to form a hereto ring; and X is a negative ion)

Formul (II)

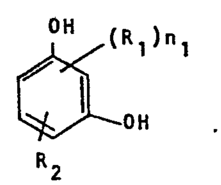

Wherein $R_1$ is a hydrogen atom, a hydroxyl group, the same or different halogen atoms, an alkyl group which may have a substituent, an aralkyl group which may have a substituent, an alkoxy group which may have a substituent, an acyl group which may have a substituent, a carboxymethyl group which may have a substituent, $-COOM$ or $-SO_3M$ group, (wherein M is a hydrogen atom, an alkali metal atom or an ammonium group); $R_2$ is a $-COOM$ or $SO_3M$ group, (wherein M is as defined above); and n is an integer of 1, 2, or 3.)

Formula (III)

./...

EP 0 089 230 A1

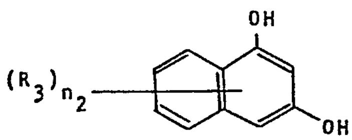

(wherein $R_3$ has the same meaning as $R_2$ defined above; and $n_2$ is the same as $n_1$ defined above)

Formula (IV)

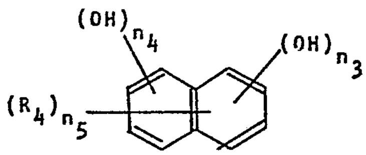

(wherein R4 has the same meaning as $R_2$ defined above; and $n_3$ and $n_4$ each is an integer of 0 or 1 but they are not both 0, and $n_5$ is an integer of 1 or 2.

# SILVER HALIDE PHOTOGRAPHIC MATERIAL

## FIELD OF THE INVENTION

The present invention relates to a silver halide photographic material that is designed to produce an image having a minimum quality deterioration and reduced fog.

## BACKGROUND OF THE INVENTION

Recent versions of silver halide photographic materials use more silver than gelatin to improve the photographic characteristics and to make it more compatible with processing steps such as development and fixation. The development step is usually performed rapidly (within 20 to 50 seconds) at high pHs under elevated temperatures (30 to 40°C), and this often causes troubles such as the shedding of layers or scrathing, producing only a photographic image of deteriorated quality.

In the manufacture of silver halide photographic materials, there is an increasing demand for shortening the period of drying the silver halide emulsion layer formed on the base. This demand for a shorter drying period or faster coating operation makes drying at low humidities unavoidable. The silver halide photographic material dried quickly at low humidities has a lower degree of hardening than the material dried by the conventional method, and when it is subjected to the subsequent rapid development at elevated temperatures, a photographic image of greatly impaired quality is formed. Many studies have been made on the method of producing silver halide photographic materials that withstand severe conditions employed for rapid processing at elevated temperatures.

One typical method is to use a "hardener" for hardening gelatin used as a binder in the photographic material. Known hardeners are inorganic compounds such as chrome alum, as well as organic compounds such as aldehyde compounds (e.g. formaldehyde and glutaraldehyde), compounds having active halogens as described in U.S. Patent No. 3,288,755, compounds having reactive ethylenically unsatrated bonds as described in U.S. Patent No. 3,635, 718, epoxy compounds of the type described in U. S. Patent No. 3,091,537, and halogen carboxyaldehydes such as mucochloric acid. But if these hardeners are incorporated in the silver halide photographic material in the amount necessary for making it resistant to rapid processing at increased temperatures, they cause adverse effects on the properties of the photographic material by increasing the fog, decreasing the sensitivity, varying the gradation or reducing the maximum density, or their hardening power changes with time to cause "after-hardening". Another method for preparing a silver halide photographic material capable of producing a quality image after rapid processing under elevated temperatures is to incorporate more gelatin (binder)=with respect to the amount of silver in the photographic material, but this method either reduces the sensitivity or renders the photographic material less compatible with development, fixing or washing procedures. Therefore, it has long been desired to devise a technique for preparing a silver halide photographic material that forms a high-quality image after repid processing at elevated temperatures without increasing the amount of gelatin (binder) with respect to the

silver content.

## SUMMARY OF THE INVENTION

An object of the present invention is to provide a silver halide photographic material that is free from the defects of the conventional product and which withstands image-forming processing under severe conditions such as repid development at elevated temperatures.

These objects of the present invention can be accomplished by a silver halide photographic material that contains in one or more of the constituent layers of the photographic material at least one compound of the following formula (I) and at least one compound selected from amoung compounds of the following formulas (II), (III) and (IV) :

Formula (I)

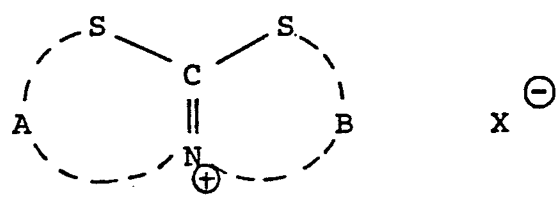

Formula (II)

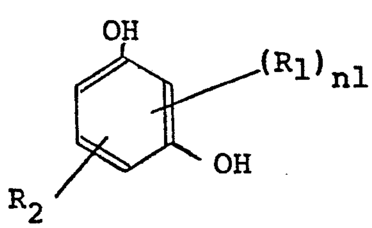

Formula (III)

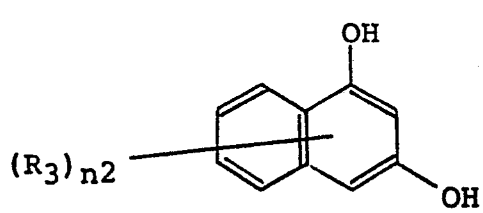

Formula (IV).

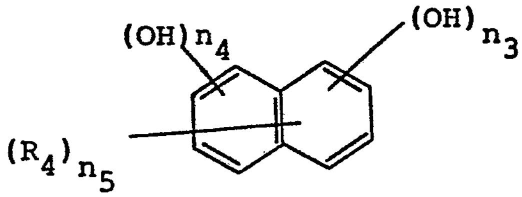

Wherein A and B are each a group of non-metallic atoms necessary to form a hereto ring; X is a negative ion (e.g. $Cl^-$, $Br^-$, $ClO_4^-$ or $CH_3SO_3^-$), $R_1$ is a hydrogen atom, a hydroxyl group, the same or different halogen atoms, an alkyl group which may have a substituent, an aralkyl group which may have a substituent, an alkoxy group which may have a substituent, an acyl group which may have a substituent, a carboxymethyl group which may have a substituent, -COOM or $-SO_3M$ group (M is a hydrogen atom, an alkali metal atom or an ammonium group); $R_2$, $R_3$ and $R_4$ are each a -COOM or $-SO_3M$ group (M is a hydrogen atom, an alkali metal atom or an ammonium group); $n_1$ and $n_2$ are each 1, 2 or 3; $n_5$ is 1 or 2; and $n_3$ and $n_4$ are 0 or 1 but they are not both 0.

The present invention is characterized by incorporating a compound of formula (I) and at least one compound which is represented by formulae (II), (III) and (IV) in combination in a silver halide photographic material. At least one compound of each type may be incorporated in at least one constituent layer, or the two compounds may be incorporated separately in two or more constituent layers on the same side of the support on which the silver halide emulsion layers are formed. The object of the present invention can be achieved in either way. In a preferred embodiment,

the compound of formula (I) (hereunder referred to as compound (A) ) is added to a silver halide emulsion layers and at least one compound represented by formulae (II), (III) and (IV) is incorporated in the same emulsion layer and/or a non-sensitive layer.

DETAILED DESCRIPTION OF THE INVENTION

Compounds (A) used in the silver halide photographic material of the present invention are described in Japanese Patent Publication Nos. 41095/72, 39169/73 and 48172/74 as compounds capable of hardening protein, forming a gelatin hardened image, and hardening vinyl polymers. If compunds (A) are contained in a silver halide photographic material in such an amount that they exhibit the hardening effect (i.e. when they are used as binders), the sensitivity of the photographic material is decreased or its gradation properties impaired. On the other hand, if these compounds are used in such an amount that they do not exhibit the hardening effect (i.e. if they are not used as binders), the decrease in the sensitivity and gradation properties of the photographic material can be prevented, but then the material is prone to cause heavy fogging.

Compounds (B) also used in the present invention are known as stabilizers for inhibiting the occurrence of fog in silver halide photographic materials, but is the photographic material containing these compounds is subjected to rapid processing under severe conditions such as elevated temperatures, only a low-quality image is produced.

Therefore, compound (A) and (B) have conflicting properties

but when they are contained in a silver photographic material in combination, it is given good photographic characteristics in that it produces a quality image after rapid processing under elevated temperatures without increasing the degree of binder hardening (i.e. reducing the sensitivity) or experiencing increased fog.

Preferred compunds (A) are such that groups A and/or B of non-metallic atoms necessary to form a hetero ring are represented by 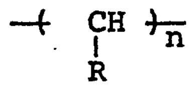 wherein R is a hydrogen atom or a lower alkyl group; n is 2 or 3.

Compounds (A) to be used in the present invention are most preferable in the case where non-metallic atoms A necessary to form a hetero rings have the following formula :

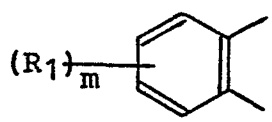

wherein $R_1$ is a hydroxyl group, a halogen atom, an alkyl group which may have a substituent, an aralkyl group which may have a substituent, an acyl group which may have a substituent, a carboxymethyl group which may have a substituent, or a -COOH or $-SO_3H$ group ; and m is an integer of 0 to 2, and non-metallic atoms B have the following formula :

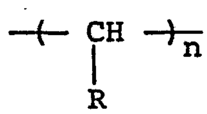

wherein R is a hydrogen atom or a lower alkyl group ; and n is an integer of 2 or 3.

Typical examples of compounds (A) that can be used in the present invention are listed below.

(A - 1)

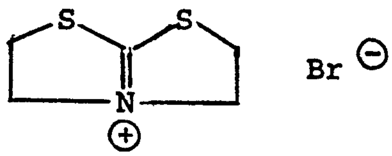

(A - 2)

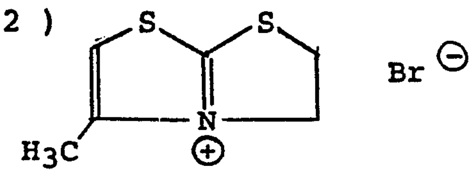

(A - 3)

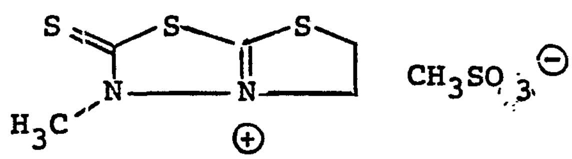

(A - 4)

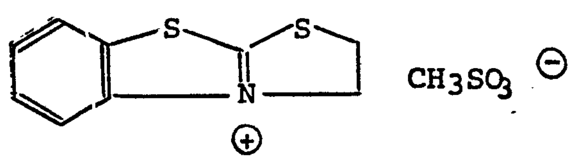

(A - 5)

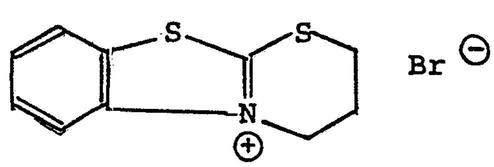

(A - 6)

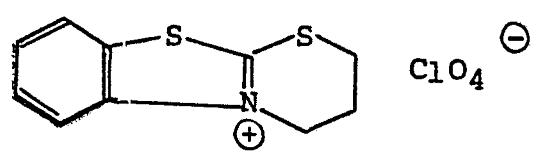

(A - 7)

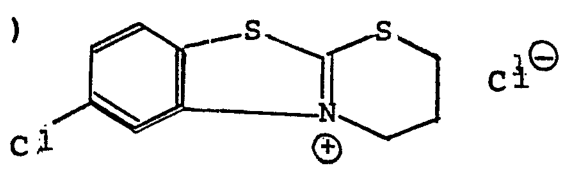

Compounds (A) can be easily synthesized from azole compounds having a methyl mercapto group, as illusted below.

Synthesis of compound (A-5)

A mixture of 2-methylthiobenzothiazole (16 g) and 1,3-dibromopropane (25 g) was heated at 160°C for 4 hours. After cooling, the resulting crystal was filtered off and recrystallized from ethanol to produce an acicular crystal of the end compound (14 g) having a melting point of 260°c.

Synthesis of compound (A-7)

Compound (A-7) can be synthesized by the method described in Chemical Abstracts, 72, 31666 (1970).

The amount of compounds (A) to be used in the present invention varies with the type of binder (e.g. gelatin), the drying conditions used in their preparation, and the composition of silver halide in the specific emulsion, but preferably, they are used in an amount of about 0.001 to 2 mg per gram of the total binder present in the hydrophilic colloidal layer on the same side of the base as the photosensitive silver halide emulsion layers. The amount of 0.01 to 1 mg is particularly preferred, and the compounds are generally used as a 0.1 to 1% solution in water or a solvent such as methanol. Compounds (A) may be added in any step prior to the coating and drying steps in the process of preparing the photographic material.

Illustrative compounds (B) of formulae (II), (III) and (IV) for use in the present invention are listed below.

( 1 )

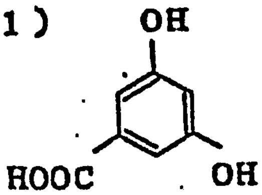

( 2 )

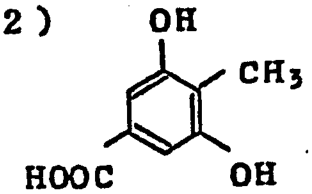

( 3 )

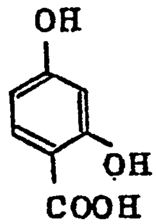

( 4 )

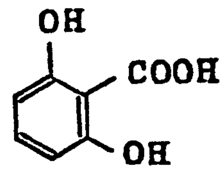

( 5 )

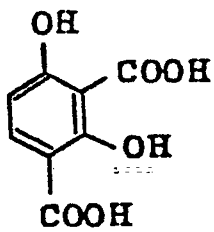

( 6 )

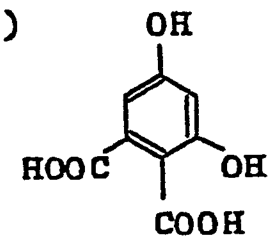

( 7 )

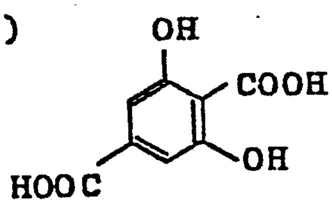

( 8 )

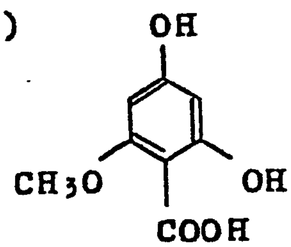

( 9 )

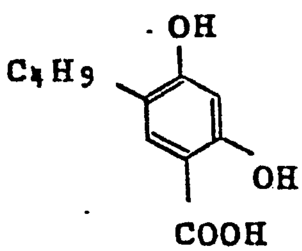

(10)

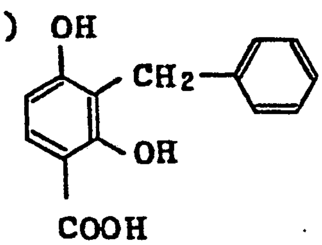

(11)

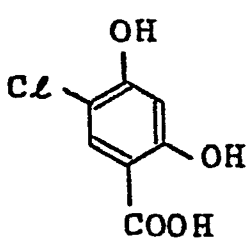

(12)

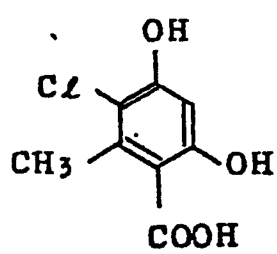

(13)

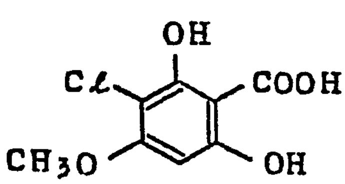

(14)

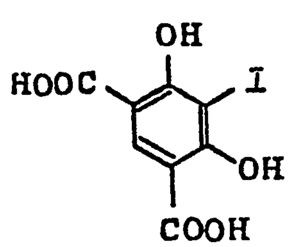

(15)

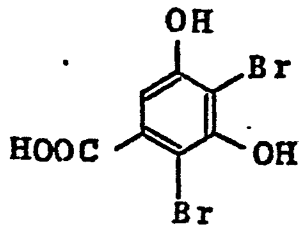

(16)

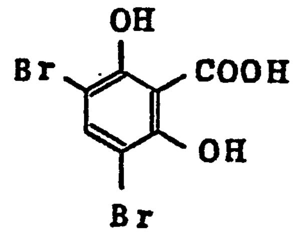

(17)

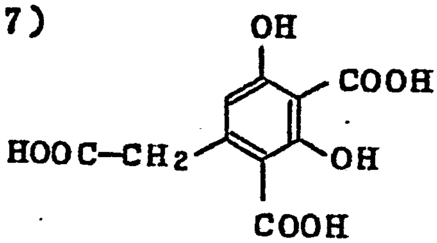

(18)

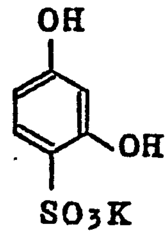

(19)

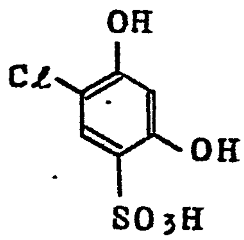

(20)

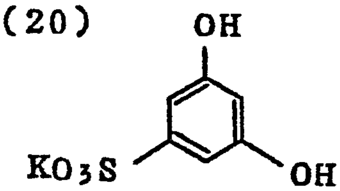

(21)

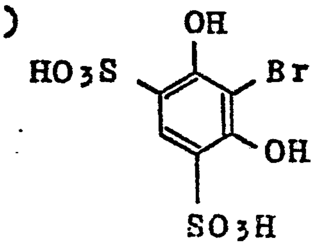

(22)

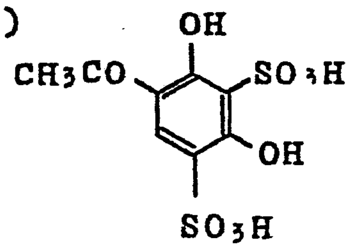

(23)

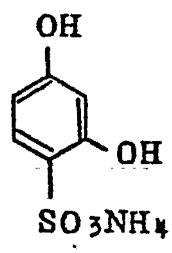

(24)

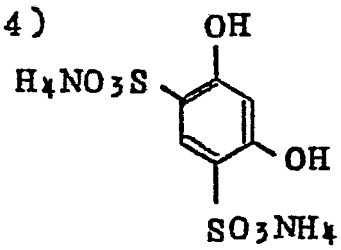

(25)

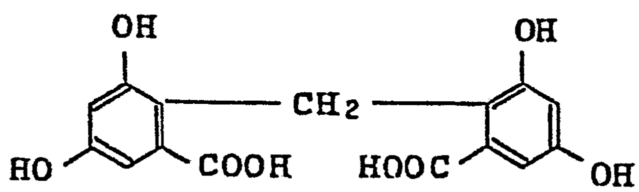

(26)

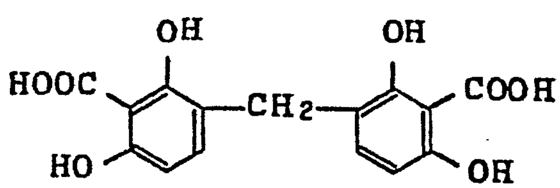

(27)

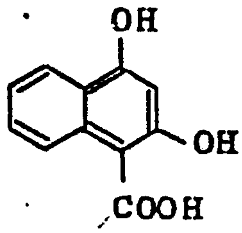

(28)

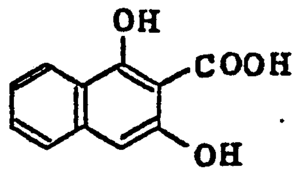

(29)

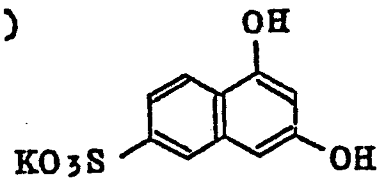

(30)

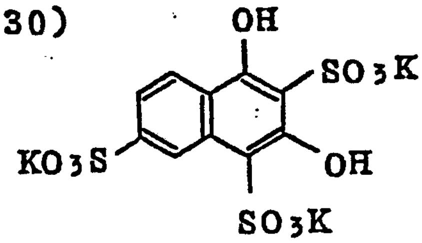

(31)

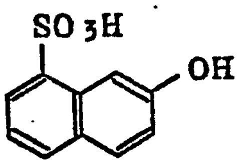

(32)

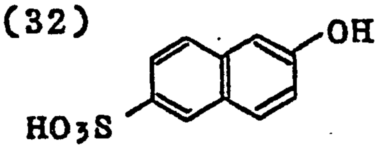

(33)

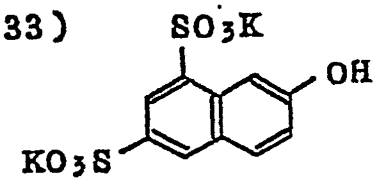

(34)

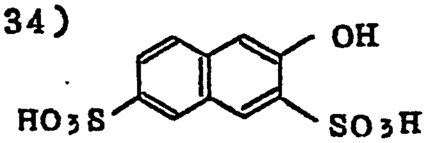

(35)

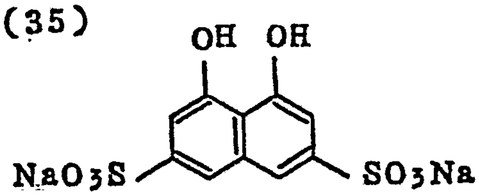

(36)

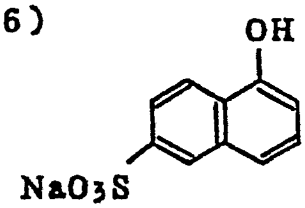

(37)

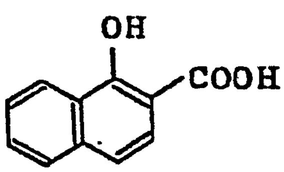

(38)

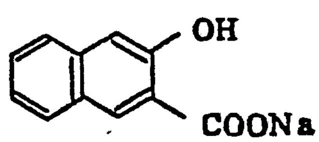

(39)

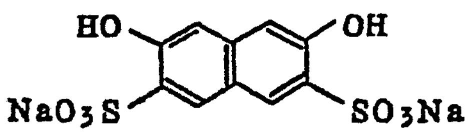

(40)

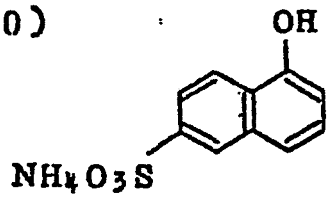

These compounds (B) can be readily synthesized by any of the methods described in Gazzetta Chimica Italiana, 57, pp. 793-802, 1927; Chemical Abstracts, 41, p. 5495, 1947; Helvetica Chimica Acta., 43, p. 644, 1960; Beilstein Organishe Chemie, 11, p. 304 and 6, p. 978; and U.S. Patent No. 2,487,586. The compounds may also be available from the commerical market.

The amount of compounds (B) to be used in the present invention varies with their type and that of the silver halide emulsion used, but usually, they are added in an amount of 0.1 to 100 g, preferably from 0.5 to 50 g, per mol of the silver halide. In most cases, the compounds are used as a 1 to 50% solution in water or a solvent such as methanol. They may be added at any stage prior to the coating step in the process of the preparation of the silver halide photographic material.

Illustrative silver halides that can be incorporated in silver halide photographic material of the present invention are silver chloride, silver bromide, silver iodide, silver chlorobromide, silver iodobromide, silver chloroiodobromide and mixtures thereof; silver iodobromide is most preferred.

Gelatin is most preferred as a binder or hydrophilic colloid for use in the present invention, but if necessary,

gelatin may be used together with gelatin derivatives, colloidal albumin, agar, gum arabic, alginic acid, cellulose derivatives, acrylamide, imidated polyacrylamide, casein, and polymers such as vinyl alcohol polymers, polyvinyl alcohol, polyvinyl pyrrolidone and hydrolyzed polyvinyl acetate.

The silver halide photographic emulsion used in the present invention may contain any of the known photographic addenda such as chemical sensitizers, spectral sensitizers, antifoggants, hard contrasting agents, gelatin hardeners, surfactants, film properties modifying agents, thickeners and halftone dot modifying agents. Illustrative chemical sensitizers include activated gelatin; noble metal sensitizers such as water soluble gold salts, platinum salts, palladium salts, rhodium salts and iridium salts; sulfur sensitizers; selenium sensitizers; and reduction sensitizers such as polyamine and stannous chloride. These chemical sensitizers may be used either alone or in combination. The spectral sensitizers that can be added are not limited to any particular compounds, and common spectral sensitizers such as cyanine or merocyanin dyes like zero-, mono-, di- and tri-methine dyes may be used either individually or in mixture for optical sensitization. For details of the spectral sensitizers, see various prior art references such as U.S. Patents Nos. 2,688,545, 2,912,329, 3,397,060, 3,615,635, and 3,628,964; British Patents Nos. 1,195,302, 1,242,588 and 1,293,862; German Patent Applications (OLS) Nos. 2,030,326 and 2,121,780; and Japanese Patent Publications Nos. 4936/68

and 14030/69.. Suitable sensitizers may be selected depending upon the region of wavelengths for which the emulsion is sensitized, as well as the sensitivity and other factors of the photographic material.

The emulsion for use in the silver halide photographic material of the present invention may contain other additives such as stabilizers or antifoggants (e.g. azaindenes, triazoles, tetrazoles, imidozolium salts, tetrazolium salts and polyhydroxy compounds); hardeners (e.g. aldehyde, aziridine, isoxazole, vinyl sulfone, acryloyl, carbodiimide, maleimide, methanesulfonate ester and triazine compounds); development accelerators (e.g. benzyl alcohol and polyoxyethylene compounds); image stabilizers (e.g. chroman, coumaran, bisphenol and phosphorous acid ester compounds); lubricants (e.g. wax, glyceride of higher aliphatic acids, and higher alcohol esters of higher aliphatic acids). Surfactants may be used as coating aids, agents to improve the permeability to processing solutions, defoamers or agents to control various physical properties of the photographic material, and any of the cationic, anionic, nonionic or amphoteric surfactants may be employed. Suitable antistats are diacetyl cellulose, styrene-perfluoroalkyl lithium maleate copolymers, and alkali salts of the reaction product of styrene-maleic anhydride copolymer and p-aminobenzenesulfonic acid. A latex may be added to provide better film properties, and suitable examples are copolymers of acrylate esters or vinyl esters and other ethylene-containing monomers. Illustrative gelatin plasticizers

include glycerin and glycolic compounds, and suitable thickeners are styrene-sodium maleate copolymers and alkylvinylether-maleic acid copolymers.

The silver halide photographic material of the present invention is formed by coating silver halide photographic emulsions onto bases optionally through subbing layers or intermediate layers, and any known method may be used to apply the silver halide emulsions. Suitable bases include baryta paper, polyethylene-coated paper, synthetic polypropylene paper, glassine paper, cellulose acetate, cellulose nitrate, polyvinylacetal, polypropylene, polyesters such as polyethylene terephthalate, and polystyrene.

The silver halide photographic material of the present invention may be used in either black-and-white or color photography. It may be used for general purposes, for printing, as well as for X-ray and other radiographic applications, and particularly significant advantages are accomplished when the material is used as a high-sensitivity silver bromide photographic material. The silver halide photographic material of the present invention may be subjected to not only ordinary exposure but also to short-time or flash exposure, and it may be processed by the conventional method of photographic processing. Two basis steps are development and fixing, which may be effected simultaneously.

The present invention is hereunder described in greater detail by reference to illustrative examples to which the scope of the invention is by no means limited.

Example 1

A high-sensitivity negative emulsion 1 kg of which contained 40 g of silver iodobromide (containing 3.5 mol% silver iodide) and 45 g of gelatin and which had been subjected to sulfur and gold sensitizations was heated at 40°C and divided into 19 portions.

(A) and (B) were added in the amounts listed in Table 1. The resulting coating solutions were applied to cellulose triacetate film bases to give a thickness 4 μ and dried. The melting points of the respective samples were measured in an aqueous solution of 0.2 N sodium hydroxide. Then, the samples were exposed to a white light in a Model KS-1 sensitometer product of Konishiroku Photo Industry Co., Ltd. by the method specified in JIS, and processed by a continuous roller transport type automatic processor that effected development, fixing, washing and drying in sequence. The development period was 30 seconds at 35°C and the developing solution used had the following formulation.

Developing solution

| | |
|---|---|
| Anhydrous sodium sulfite | 70 g |
| Hydroquinone | 10 g |
| Boric anhydride | 1 g |
| Sodium carbonate (monohydrate) | 20 g |
| 1-Phenyl-1,3-pyrazolidone | 0.35 g |
| Sodium hydroxide | 5 g |
| 5-Methyl benzotriazole | 0.05 g |
| Potassium bromide | 5 g |
| Glutaraldehyde bisulfite | 15 g |
| Glacital acetic acid | 8 g |

Water to make 1,000 ml

The so processed samples were subjected to sensitometry to determine their photographic characteristics which are shown in Table 1 wherein the sensitivity is indicated as relative values with that of control (Sample No. 1) being taken as 100. The image quality was evaluated by visually inspecting the deterioration of developed silver grains at a density of 0.8. The criteria were ○ (good), Δ (moderate) and × (very poor). The combination of two of these symbols indicate the state in between those represented by the respective symbols.

Table 1

| Sample | Compound A | | Compound B | | pH of Emulsion | Melting point (°C) | Photographic Characteristics | | | | Image Quality |
|---|---|---|---|---|---|---|---|---|---|---|---|
| | | | | | | | After standing 3 days at 25°C and 60% r.h. (without heat treatment | | After standing 3 days at 50°C and 50°C and 60% r.h. | | |
| | Identification No. | Amount (mg/100g-emulsion) | Identification No. | Amount (mg/100g-emulsion) | | | Fog | Sensitivity | Fog | Sensitivity | |
| 1 | - | - | - | - | 6.5 | 28 | 0.03 | 100 | 0.05 | 100 | △ |
| 2 | A-1 | 4.5 | - | - | " | " | 0.04 | 98 | 0.16 | 90 | ○ △ |
| 3 | A-2 | 4.5 | - | - | " | " | 0.04 | 98 | 0.18 | 95 | ○ △ |
| 4 | A-3 | 4.5 | - | - | " | " | 0.03 | 98 | 0.20 | 95 | ○ △ |
| 5 | A-4 | 4.5 | - | - | " | " | 0.04 | 98 | 0.24 | 95 | ○ △ |
| 6 | A-5 | 0.45 | - | - | " | " | 0.04 | 98 | 0.18 | 95 | ○ △ |
| 7 | " | 4.5 | - | - | " | " | 0.04 | 98 | 0.24 | 90 | ○ △ |
| 8 | " | 45 | - | - | " | 33 | 0.04 | 82 | 0.16 | 75 | ○ △ |
| 9 | " | 450 | - | - | " | 38 | 0.02 | 65 | 0.08 | 52 | ○ |
| 10 | A-6 | 4.5 | - | - | " | 28 | 0.04 | 95 | 0.18 | 90 | ○ △ |
| 11 | A-7 | 4.5 | - | - | " | " | 0.04 | 98 | 0.20 | 95 | ○ △ |
| 12 | - | - | (10) | 100 | " | " | 0.02 | 100 | 0.03 | 102 | × |
| 13 | - | - | (20) | 10 | " | " | 0.02 | 102 | 0.04 | 105 | × |
| 14 | - | - | " | 100 | " | " | 0.02 | 100 | 0.03 | 102 | × |
| 15 | - | - | (30) | 100 | " | " | 0.02 | 100 | 0.03 | 105 | × |
| 16 | - | - | (40) | 100 | " | " | 0.02 | 100 | 0.03 | 105 | × |
| 17 | A-2 | 4.5 | (20) | 100 | " | " | 0.02 | 100 | 0.04 | 105 | ○ △ |
| 18 | A-5 | 0.45 | " | 100 | " | " | 0.03 | 100 | 0.04 | 105 | ○ △ |
| 19 | " | 4.5 | " | 100 | " | " | 0.03 | 100 | 0.05 | 100 | ○ △ |

As Table 1 show, the samples according to the present invention had high sensitivities and produced good images with minimum fog. But the samples outside the scope of the present invention were defective with respect to at least one of the three factors, fog, sensitivity and image quality. Otherwise, they had increased melting points.

Example 2

An X-ray silver iodobromide emulsion in gelatin that contained 2 mol% of silver iodide was subjected to the second ripening and mixed with saponin as a coating aid. The mixture was then divided into 20 portions. Compounds (A) and compounds (B) were added in the amounts indicated in Table 2. The resulting coating solutions were applied onto polyethylene terephthalate film bases to give a dry thickness of 5 μ and dried. The respective samples were subjected to X-ray sensitometry through a medium-sensitivity fluorescent intensifying screen (product of Toshiba Corp.) and an aluminum step wedge at a tube voltage of 60 kVp and a tube current of 200 mA. The samples were then processed as in Example 1 to evaluate their sensitivity and the degree of fog, and the results are shown in Table 2. At the same time, the melting points of the samples were measured as in Example 1 to evaluate the quality of the resulting images. The results are also shown in Table 2 .

Table 2

| Sample No. | Compound A | | Compound B | | Melting Point (°C) | Photographic Characteristics | | | | Image Quality |
|---|---|---|---|---|---|---|---|---|---|---|
| | Identification No. | Amount (mg/ gelatin) | Identification No. | Amount (mg/ 1 mol- Silver halide) | | After standing 3 days at 25°C and 65% r.h. (without heat treatment) | | After standing 3 days at 50°C and 60% r.h. | | |
| | | | | | | Fog | Sensitivity | Fog | Sensitivity | |
| 1 | – | – | – | – | 30 | 0.04 | 100 | 0.06 | 110 | △ |
| 2 | A-6 | 0.5 | – | – | 30 | 0.04 | 95 | 0.18 | 100 | ○ △ |
| 3 | " | 10 | – | – | 36 | 0.04 | 80 | 0.14 | 60 | ○ △ |
| 4 | H-1 * | 1 | – | – | 36 | 0.04 | 98 | 0.07 | 105 | △ |
| 5 | " | 5 | – | – | 50 | 0.03 | 90 | 0.06 | 85 | ○ △ |
| 6 | H-2 | 3 | – | – | 42 | 0.04 | 95 | 0.08 | 85 | ○ △ |
| 7 | – | – | (18) | 1.0 | 27 | 0.04 | 100 | 0.05 | 110 | △ X |
| 8 | – | – | " | 10 | " | 0.04 | 100 | 0.04 | 110 | △ X |
| 9 | – | – | B-1 | 0.005 | 30 | 0.03 | 98 | 0.04 | 105 | △ |
| 10 | – | – | " | 0.05 | " | 0.02 | 60 | 0.03 | 65 | △ |
| 11 | – | – | B-2 | 0.005 | " | 0.03 | 95 | 0.04 | 90 | △ |
| 12 | A-6 | 0.5 | (18) | 1.0 | 27 | 0.04 | 100 | 0.05 | 110 | ○ |
| 13 | " | " | B-1 | 0.005 | 30 | 0.03 | 95 | 0.16 | 95 | ○ △ |
| 14 | " | " | B-2 | 0.005 | 30 | 0.03 | 90 | 0.15 | 80 | ○ △ |
| 15 | " | 1 | (18) | 1.0 | 27 | 0.03 | 100 | 0.04 | 110 | ○ |
| 16 | " | 5 | B-1 | 0.005 | 36 | 0.03 | 75 | 0.14 | 55 | ○ |
| 17 | " | " | B-2 | 0.005 | " | 0.03 | 70 | 0.13 | 50 | ○ |
| 18 | H-1 | 1 | (18) | 1.0 | 34 | 0.04 | 95 | 0.06 | 105 | △ |
| 19 | " | 5 | " | " | 50 | 0.03 | 90 | 0.05 | 85 | ○ △ |
| 20 | H-2 | 3 | " | " | 42 | 0.04 | 95 | 0.06 | 80 | ○ △ |

* Compounds (H-1) and (H-2) were hardeners which respectively had the following formulae:

(H-1) $(CHO)_2$

(H-2) 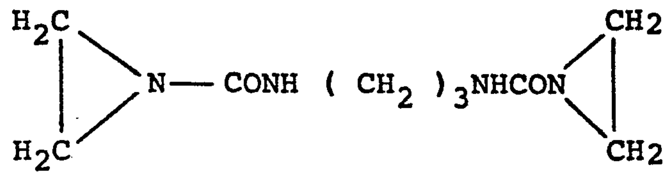

** Compounds (B-1) and (B-2) were development restrainers which respectively had the following formulae:

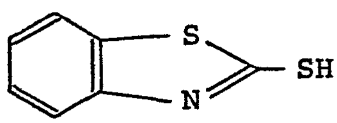

(B-1)

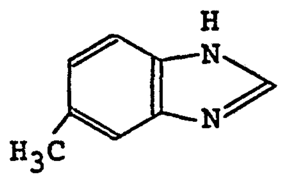

(B-2)

As Table 2 show, the samples accoridng to the present invention had high sensitivities and produced good images with minimum fog. Furthermore, their melting points were not as high as to cause excessive hardening of the emulsion layer.

Example 3

A triacetate film base having an anti-halation layer was coated with a first layer or a layer of high-sensitivity color photographic silver iodobromide emulsion (dry thickness: 5 μ) wherein 1 kg of the negative silver halide contained 10 g of 4-chloro-1-hydroxy-2-n-octylnaphthamide as a coupler and which had been sensitized for red light, The first layer was overlaid with a second layer or a layer of 3% aqueous gelatin solution (dry thickness: 1 μ) Then, a third layer

or a layer of high-sensitivity color photographic silver iodobromide emulsion (dry thickness: 4 μ) wherein 1 kg of the negative silver halide contained 10 g of 1-(3-carboxyphenyl)-3-(4-stearoylaminophenyl)-5-pyrazolone as a coupler and which had been sensitized for green light was formed, and on that layer was formed a fourth layer or a layer of yellow colloidal silver in a dry thickness of 2 μ. Then, a fifth layer or a layer of high-sensitivity silver iodobromide emulsion (dry thickness: 7 μ) wherein 1 kg of the negative silver halide contained 13 g of 2-dodecyloxybenzoylacetanilide as a coupler and which had been sensitized for blue light was formed, and on that layer was formed a sixth layer or a layer of 4 % aqueous gelatin solution in a dry thickness of 1 μ. In this way, seven samples of coupler-in emulsion type color photographic negative film were prepared. In the second fourth and sixth layers of each sample, 3 mg of formalin was incorporated as a hardener per kg of the gelatin.

At the time of the completion of the second ripening, 4-hydroxy-6-methyl-1,3,3a,7-tetrazaindene (stabilizer) and saponin (coating aid) were added to the first, third and fifth silver halide emulsion layers. Thereafter, compounds (A) and (B) were added to the first, third and fifth layers in the amounts indicated in Table 3. The so prepared samples were left to stand at 25°C and 60% r.h. for 30 days. After processing the samples with a color developing solution (for the formulation, see below) at 24°C for 5 minutes, the color desities of the resulting images were measured with a color densitometer. The photographic characteristics determined from the color densities are also shown in Table

3, wherein the fog and sensitivity values indicated in columns R, G and B were determined from those values which were measured through red, green and blue color photographic densitometer filters, respectively, and the sensitivity was indicated in terms of relative values which the red sensitivity of sample No. 1 at 20°C and 60% r.h. being taken as 100. The development period was 5 minutes at 24°C and the developing solution used had the following formulation.

Color developer

| | |
|---|---|
| Benzyl alcohol | 3.8 ml |
| Anhydrous sodium sulfite | 2.0 g |
| N-Ethyl-N-β-methanesulfonamidoethyl-3-methyl-4-aminoaniline sulfate | 5.0 g |
| Sodium carbonate | 5.0 g |
| Sodium bromide | 1.0 g |
| Water | to make 1,000 ml |

Table 3

| Sample No. | Compound A | | Compound B | | Melting Point (°C) | Photographic Characteristics after standing 30 days at 25°C and 60% r.h. | | | | | | Imag Quali |
|---|---|---|---|---|---|---|---|---|---|---|---|---|
| | Identification No. | Amount (mg/gelatin) | Identification No. | Amount (g/1 mol Silver halide) | | Fog | | | Sensitivity | | | |
| | | | | | | R | G | B | R | G | B | |
| 1 | - | - | - | - | 52 | 0.08 | 0.08 | 0.08 | 100 | 110 | 95 | △ |
| 2 | A-4 | 0.5 | - | - | " | 0.15 | 0.14 | 0.17 | 80 | 85 | 75 | ○ |
| 3 | - | - | (18) | 1.5 | 50 | 0.07 | 0.07 | 0.07 | 100 | 110 | 95 | △ > |
| 4 | A-4 | 0.5 | " | " | " | 0.07 | 0.08 | 0.07 | 100 | 110 | 95 | ○ |
| 5 | Ⓐ * | 2 | " | " | 58 | 0.07 | 0.08 | 0.08 | 98 | 105 | 90 | △ |
| 6 | " | 5 | " | " | 75 | θ.10 | 0.09 | 0.09 | 75 | 75 | 70 | ○ |
| 7 | A-4 | 0.5 | Ⓑ ** | 0.005 | 52 | 0.07 | 0.06 | 0.07 | 45 | 105 | 95 | △ |

* Ⓐ denotes a hardener 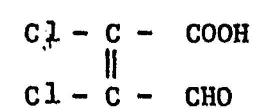

** Ⓑ denotes a Inhibitor 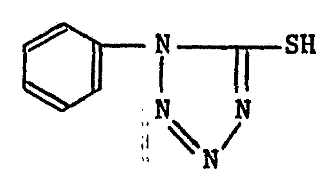

As Table 3 shows, sample No. 4 according to the present invention had a high sensitivity and produced a good image with minimum fog.

0089230



C L A I M S

1. A silver halide photographic material comprising a support and one or more layers, at least one layer containing a compound of the following formula

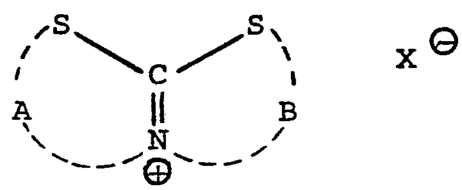

(I)

wherein A and B each independently represents the non-metallic atoms necessary to form a hetero ring; and X is a negative ion and at least one layer containing a compound of the following formulae (II), (III), or (IV):

Formula (II)

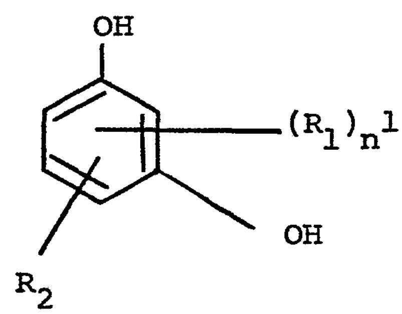

wherein the or each $R_1$ is independently a hydrogen atom, a hydroxyl group, a halogen atom, an alkyl group which may have a substituent, an aralkyl group which may have a substituent, an alkoxy group which may have a substituent, an acyl group which may have a substituent, a carboxymethyl group which may have a substituent, -COOM or $-SO_3M$ group wherein M is a hydrogen atom, an alkali metal atom or an ammonium group; $R_2$ is a -COOM or $-SO_3M$ group wherein M is as defined above; and $n^1$ is 1, 2 or 3.

Formula (III)

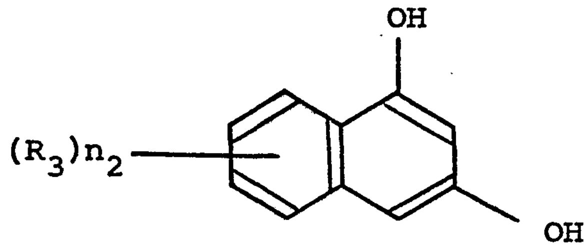

wherein $R_3$ has the same meaning as $R_2$ defined above; and $n_2$ is the same as $n_1$ defined above.

Formula (IV)

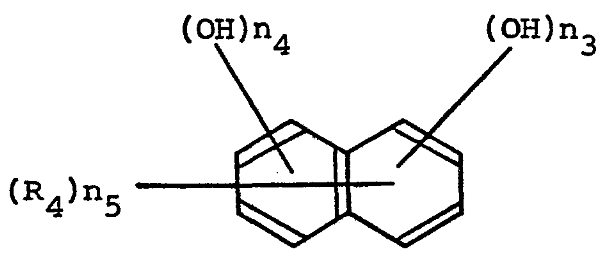

wherein $R_4$ has the same meaning as $R_2$ defined above; and $n_3$ and $n_4$ each independently is 0 or 1 such that they are not both 0, and $n_5$ is 1 or 2.

2. A silver halide photographic material according to claim 1, wherein said A or B in formula (I) has the formula

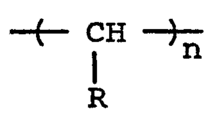

wherein n is 2 or 3; and R is a hydrogen atom or a lower alkyl group generally having 1 to 6 carbon atoms.

3. A silver halide photographic material according to claim 2 wherein n is 2.

4. A silver halide photographic material according to claim 1 wherein said A or B in formula (I) has the following formula:

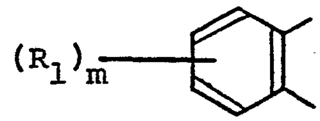

wherein $R_1$ is a hydroxyl group, a halogen atom, an alkyl group which may have a substituent, an aralkyl group which may have a substituent, an acyl group which may have a substituent, a carboxymethyl group which may have a substituent, or a $-COOH$ or $-SO_3H$ group; and m is 0, 1 or 2.

5. A silver halide photographic material according to claim 4 wherein said A or B in formula (I) has the formula

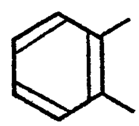

6. A silver halide photographic material according to claim 1 wherein said A in formula (I) has the formula

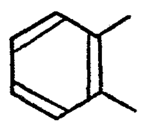

and said B in formula (I) has the formula

$$-(CH_2)_2- \quad .$$

7. A silver halide photographic material according to any one of claims 1 to 6 wherein said compound of formula (I) is present in an amount from 0.001 to 2 mg per gram of total binder present in a hydrophilic colloidal layer.

8. A silver halide photographic material according to any one of claims 1 to 7 wherein at least one compound of formula (II), (III), or (IV) is present in an amount of 0.1 to 100 g per mol of the silver halide.

9. A silver halide photographic material according to any one of claims 1 to 8 which is suitable for X ray use.

European Patent Office

# EUROPEAN SEARCH REPORT

0089230

Application number

EP 83301426.9

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| A | DE - A - 2 133 053 (FUJI)<br>* Claims 1-7, 9-13; page 7, lines 5-9; page 8, lines 1-13; page 8, line 29 - page 9, line 24 *<br>-- | 1-7,9 | G 03 C 1/06<br>G 03 C 1/30<br>G 03 C 1/34<br>G 03 C 5/16<br>C 07 D 513/02 |
| A,D | DE - A - 2 151 095 (FUJI)<br>* Claims 1-3, 6-9, 11-13, 15; page 6, lines 8-22 *<br>& JP-B4-48-039 169 (1973)<br>-- | 1-6 | |
| A,D | US - A - 3 773 731 (OHI)<br>* Claims 1, 3-7, 9-16 *<br>& JP-B4-49-48 172 (1974)<br>-- | 1-6 | |
| A | DE - A1 - 2 422 772 (KONISHIROKU)<br>* Claims 1, 3-5; page 21-23; example 2 *<br>---- | 1,8,9 | TECHNICAL FIELDS SEARCHED (Int. Cl. 3)<br>G 03 C<br>C 07 D 513/00 |

The present search report has been drawn up for all claims

| Place of search | Date of completion of the search | Examiner |
|---|---|---|
| VIENNA | 15-06-1983 | SCHÄFER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82